# EUROPEAN PATENT SPECIFICATION

(11) **EP 4 572 669 B1**
(45) Date of publication and mention of the grant of the patent: **05.11.2025**
(21) Application number: 23783608.5
(22) Date of filing: 12.09.2023
(51) Int. Cl.: A61M 39/20, A61B 5/15, A61B 5/153, A61M 39/06, A61B 5/154, A61M 39/26

(54) **FLUID SHIELD ASSEMBLIES AND FLOW RESTRICTION DEVICES WITH FLUID SHIELD ASSEMBLIES**
FLÜSSIGKEITSSCHILDANORDNUNGEN UND STRÖMUNGSBEGRENZUNGSVORRICHTUNGEN MIT FLÜSSIGKEITSSCHILDANORDNUNGEN
ENSEMBLES DE PROTECTION CONTRE LES FLUIDES ET DISPOSITIFS DE RESTRICTION DE DÉBIT AVEC ENSEMBLES DE PROTECTION CONTRE LES FLUIDES

(30) Priority: 27.09.2022 US 202217953892
(43) Date of publication of application: 25.06.2025
(73) Proprietor: CareFusion 303, Inc., San Diego, CA 92130 (US)
(72) Inventor: SALEHI BOROJERDI, Alireza, San Diego, California 92130 (US); KHAN, Mohammed Mehtab, Karnataka, Bengaluru 560045 (IN); LEKKALA, Niranjan, Karnataka, Bengaluru 560045 (IN); MUNDADISHETTY, Sudharshan, Karnataka, Bengaluru 560045 (IN); KAKARAPARTHI, Prabhath, Karnataka, Bengaluru 560045 (IN)
(74) Representative: Zenz Patentanwälte Partnerschaft mbB
(86) International application number: PCT/US2023/032535
(87) International publication number: WO 2024/072626

(56) References cited:
- WO-A1-2010/028040
- WO-A1-2018/025094
- WO-A1-95/32748
- US-A- 5 104 389
- US-A1- 2021 068 729
- US-A1- 2022 110 562

## Description

### TECHNICAL FIELD

The present invention relates to a fluid shield assembly according to the preamble of claim 1. Such a fluid shield assembly is known from WO 95/32748 A1.

The present disclosure generally relates to intravenous (IV) blood draw from a patient, and particularly to systems to reduce hemolysis in an over-the-needle peripheral IV catheter (PIVC) blood draw whilst reducing blood spillage.

### BACKGROUND

Catheters are commonly used for a variety of infusion therapies. For example, catheters may be used for infusing fluids, such as normal saline solution, various medicaments, and total parenteral nutrition, into a patient. Catheters may also be used for withdrawing blood from the patient.

A common type of catheter is a PIVC. As its name implies, the over-the-needle catheter may be mounted over an introducer needle having a sharp distal tip. A catheter assembly may include a catheter hub, the catheter extending distally from the catheter hub, and the introducer needle extending through the catheter. The catheter and the introducer needle may be assembled so that the distal tip of the introducer needle extends beyond the distal tip of the catheter with the bevel of the needle facing up away from skin of the patient. The catheter and introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of the catheter assembly. Once placement of the needle has been confirmed, the clinician may temporarily occlude flow in the vasculature and remove the needle, leaving the catheter in place for future blood withdrawal or fluid infusion.

For blood withdrawal or collecting a blood sample from a patient, a blood collection container may be used. The blood collection container may include a syringe. Alternatively, the blood collection container may include a test tube with a rubber stopper at one end. In some instances, the test tube has had all or a portion of air removed from the test tube so pressure within the test tube is lower than ambient pressure. Such a blood collection container is often referred to as an internal vacuum or a vacuum tube. A commonly used blood collection container is a VACUTAINER^{®} blood collection tube, available from Becton Dickinson & Company.

The blood collection container may be coupled to the catheter. When the blood collection container is coupled to the catheter, a pressure in the vein is higher than a pressure in the blood collection container, which pushes blood into the blood collection container, thus filling the blood collection container with blood. A vacuum within the blood collection container decreases as the blood collection container fills, until the pressure in the blood collection container equalizes with the pressure in the vein, and the flow of blood stops. Unfortunately, as blood is drawn into the blood collection container, red blood cells are in a high shear stress state and susceptible to hemolysis due to a high initial pressure differential between the vein and the blood collection container. Hemolysis may result in rejection and discard of a blood sample. The high initial pressure differential can also result in catheter tip collapse, vein collapse, or other complications that prevent or restrict blood from filling the blood collection container. Furthermore, blood spillage during and/or after blood draw commonly occurs.

WO 95/32748 A1 describes a self-sealing valve device for angiographic catheters. The document discloses a valve device that includes a housing with a first end adapted to form a luer lock connection with the proximal end of an angiographic catheter. A central bore extends between the first end of the housing and an opposing second end. A deformable elastomeric slit seal is supported across the central bore to selectively seal the proximal end of the catheter. The slit seal is designed to permit a guide wire to pass through while sealingly engaging the walls of the guide wire to prevent blood loss through the catheter. The second end of the housing features a female luer lock connection fitting for receiving the conical tip of a syringe, stopcock, or similar device, and forming a pressure tight connection between them. The valve device allows for the introduction of guide wires and the formation of secure connections with other medical equipment while maintaining a seal to prevent blood loss.

### SUMMARY

The invention is defined in the independent claim 1. Preferable embodiments are further laid out in the dependent claims. In one or more embodiments, a flow restriction device, comprises: a proximal housing configured to couple to a fluid collection device; a distal housing configured to couple to a catheter assembly; an intermediate housing interposed between the proximal and distal housings; an internal fluid channel extending transversely through the proximal, intermediate and distal housings; and a fluid shield assembly. The fluid shield assembly comprises an enclosure coupled to an end of the distal housing and a split septum coupled to an end of the enclosure, the split septum comprising a flexible material with a centrally disposed slit. The fluid shield assembly is configured to allow connection and disconnection of a female connector to a male connector of the distal housing through the slit of the split septum, and to contain fluid within the enclosure and split septum upon disconnection and withdrawal of the female connector from the male connector.

In one or more embodiments, a blood collection system comprises: a blood collection device; a catheter assembly; and a flow restriction device fluidly coupled to the blood collection device. The flow restriction device comprises: a proximal housing coupled to a male connector of the blood collection device; a distal housing comprising a male luer connector configured to couple to a female connector of the catheter assembly; an intermediate housing interposed between the proximal housing and the distal housing; an internal fluid channel extending transversely through the proximal, intermediate and distal housings; and a fluid shield assembly. The fluid shield assembly comprises: a luer enclosure coupled to an end of the distal housing; and a split septum coupled to an end of the enclosure, the split septum comprising a flexible material with a centrally disposed slit, the split septum and the luer enclosure enclosing the male luer connector and an end portion of the distal housing. The fluid shield assembly provides for connection and disconnection of the female connector of the catheter assembly to the male luer connector of the distal housing through the slit of the split septum, and to contain excess blood within the enclosure and split septum upon disconnection and withdrawal of the female connector from the male luer connector.

In one or more embodiments, a fluid shield assembly for a male luer connector comprises: an enclosure configured to couple to an end of the male luer connector; and a split septum coupled to an end of the enclosure, the split septum comprising a flexible material with a centrally disposed slit, wherein the fluid shield assembly is configured to provide connection and disconnection of a female connector to the male luer connector through the slit of the split septum, and to contain fluid within the enclosure and split septum upon disconnection and withdrawal of the female connector from the male luer connector.

### BRIEF DESCRIPTION OF THE DRAWINGS

The following figures are included to illustrate certain aspects of the embodiments and should not be viewed as exclusive embodiments. The subject matter disclosed is capable of considerable modifications, alterations, combinations, and equivalents in form and function, as will occur to those skilled in the art and having the benefit of this disclosure.
FIG. 1 illustrates a vascular access device including a PIVC assembly that includes a flow restriction device, in accordance with some embodiments of the present disclosure.
FIG. 2 illustrates a perspective view of the flow restriction device, in accordance with some embodiments of the present disclosure.
FIG. 3 illustrates a vascular access device including a PIVC assembly that includes a flow restriction extension set, in accordance with some embodiments of the present disclosure
FIG. 4 illustrates a perspective view of the flow restriction extension set, in accordance with some embodiments of the present disclosure.
FIG. 5 illustrates a cross-sectional perspective view of a portion of a fluid shield assembly and male luer connector, in accordance with some embodiments of the present disclosure.
FIG. 6 illustrates a top view of a fluid shield assembly, in accordance with some embodiments of the present disclosure.
FIG. 7 illustrates a bottom view of the fluid shield assembly of FIG. 6, in accordance with some embodiments of the present disclosure.
FIG. 8 illustrates a bottom perspective view of the fluid shield assembly of FIG. 6, in accordance with some embodiments of the present disclosure.
FIG. 9 illustrates a cross-sectional perspective view of the fluid shield assembly of FIG. 6, in accordance with some embodiments of the present disclosure.
FIG. 10 illustrates a perspective view of a connector assembly having a female connector coupled to a male connector and a fluid shield assembly, in accordance with some embodiments of the present disclosure.
FIG. 11 illustrates a cross-sectional perspective view of the connector assembly of FIG. 10, in accordance with some embodiments of the present disclosure.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions may be provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

Blood draw via a vascular access device has drawn increasing attention attributed to minimized needle sticks and improved operation efficiency as compared with traditional blood draw methods with venipuncture. Current blood draw using a PIVC has seen some challenges, one of the most critical is hemolysis related blood quality. In particular, with currently existing PIVC products in the market, along with the standard connection (such as a short extension set and a needleless connector), and blood collection devices (such as a Vacutainer), the shear stress exerted onto blood cells tends to be on the verge of hemolyzing.

Various embodiments of the present disclosure are directed to providing assemblies to address blood spillage in PIVC blood draw with a hemolysis reduction accessory (also referred to herein as a flow restriction device) which is pre-attached to the PIVC and serves as a flow restrictor to reduce risk of hemolysis. The hemolysis-reduction accessory is advantageously compatible with PIVC placement and does not necessitate change to any of the existing operations. The hemolysis-reduction accessory of the various embodiments described herein is potentially applicable to a wide variety of PIVC products, and compatible with existing blood collection devices and infusion disposables.

Here, the flow restriction device regulates the overall flow rate of the entire fluid path as blood cells travel through. The flow restriction device can be either assembled with the PIVC or co-packaged with the PIVC. As such, there is no additional operation during catheter placement since the device has a vented lumen that enables blood flashback. The clinician may connect a blood collection device to the port of the accessory and can then draw blood to the intended volume. After blood draw, the clinician may disconnect and discard the flow restriction device and the blood collection device together. As such, this flow restriction device can be either for single blood draw or stay inline throughout indwell. In some embodiments, the flow restriction device may be an integral connector that includes a proximal housing, a distal housing and an intermediate housing, each having an aligned fluid channel disposed axially within. Accordingly, fluid communication may be established between the proximal, distal, and intermediate housings to allow a fluid to flow from the distal housing to the proximal housing via the intermediate housing. The aligned internal fluid channels together may define a linear internal flow channel through the flow restriction device. As such, during blood collection or withdrawal from the patients, blood may flow from the patient's vein into a blood collection device via the internal flow channel of the flow restriction device.

In some embodiments, the flow restriction device may be an extension set that includes an IV tube with a male luer connection on one end and a female luer connection on the other end. Accordingly, fluid communication may be established between the male and female luer connections and the IV tube to allow a fluid to flow from the female luer connection to the male luer connection via the IV tube. As such, during blood collection or withdrawal from the patients, blood may flow from the patient's vein into a blood collection device via an internal flow channel of the flow restriction device.

During blood draw with currently existing blood draw devices, for example, blood cells may experience shear stress as they flow from the distal housing to the proximal housing. The maximum shear stress may be along the wall of the blood cell, often referred to as wall shear stress. Wall shear stress on blood cells is considered a major source of mechanical damage to blood cells causing hemolysis of the blood cells. In some embodiments, the linear internal flow channel of the flow restriction device having minimal diameter may facilitate increased flow resistance within the vascular access system to distribute the pressure differential and reduce shear stress experienced by the red blood cells of the blood. For example, the minimized diameter of the internal flow channel may provide increased resistance to flow of the blood and thereby decrease blood flow rate within the flow resistance device. Since the decreased blood flow rate causes a reduction in shear stress experienced by the red blood cells of the blood, a risk of hemolysis during blood collection may advantageously be reduced.

In some embodiments, the integral connector flow restriction device distal housing may include a male connector, such as a male luer connector, and the proximal housing may include a female connector, such as a needleless connector. In some embodiments, the extension set flow restriction device may include a male connector, such as a male luer connector, and a female connector, such as a needleless connector. In some embodiments, the flow restriction device may include a fluid shield assembly coupled to the distal housing or the male connector. For example, the fluid shield assembly may include a split septum and an enclosure to fit on the male luer connector.

Various embodiments of the present disclosure are directed to providing assemblies to address blood spillage in PIVC blood draw with an extension set with a male luer connector on one end and a female luer connector on the opposing end. A standard ISO male luer connector is used to be connected to a catheter side needle-free connector, which has the potential of blood exposure from the male luer connector while disconnecting after blood draw is complete.

A blood draw device is often used with a luer lock access device (LLAD) or a syringe (e.g., the blood draw device is connected with an LLAD or a syringe and then used for a blood draw). It is typically prescribed to dispose the LLAD or the syringe and the blood draw device together to avoid blood spillage. After the blood draw, i.e., after removal of a blood filled container (e.g., Vacutainer), the next step is to disconnect the blood draw device that is filled with remaining blood. Thus, an issue is the potential blood exposure to a clinician as the clinician disconnects the blood draw device from the PIVC needle-free connector.

In some embodiments, a split septum with a small enclosure around the male luer is provided at the front of the blood draw device connector. The needle-free connector is pushed into the split septum while it is connected to the blood draw device. The split septum self-seals and prevents or minimizes left over blood from spilling from the blood draw device when the needle-free connector is removed. The septum blocks within the enclosure and avoids any spillage. The split septum may be made of silicone material and may be coupled to the luer enclosure in any suitable manner (e.g., glued, welded, overmolded). The split septum may also be coupled to the blood draw device connector at the front of the male luer. For example, the split septum may be glued to the enclosure and the enclosure may be friction fit over the front end of the male luer connector of the blood draw device, where the split septum may also be glued to the front rim of the male luer connector.

The flow restriction devices and associated blood collection systems of the various embodiments described herein additionally provide further advantages over currently existing blood collection systems. For example, add-on flow restriction devices described herein allow for hemolysis-reduction function to be integrated for PIVC blood draw. Further, the flow restriction devices described herein are compatible with PIVC placement and allow for seamless blood draw at insertion. Additionally, since the flow restriction devices are an add-on which can be easily incorporated without any changes to existing PIVC, there is minimal impact to clinical setting and operations. As another example, extension sets with a male luer connector described herein allow for safe disconnection from an existing PIVC.

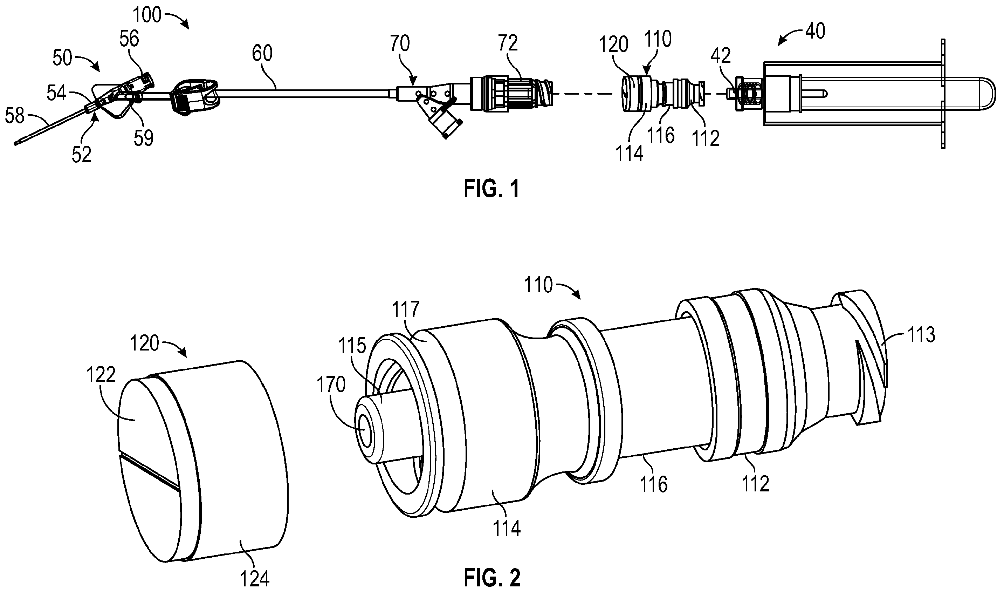

FIG. 1 illustrates a vascular access device 100 including a PIVC assembly 50 that includes a flow restriction device 110, in accordance with some embodiments of the present disclosure. The flow restriction device 110 may be configured to reduce a likelihood of hemolysis during blood collection using the vascular access device 100. In some embodiments, the vascular access device 100 may include the catheter assembly 50. The catheter assembly 50 may include a catheter hub 52, which may include a distal end 54, a proximal end 56, and a lumen extending through the distal end 54 and the proximal end 56. The catheter assembly 50 may further include a catheter 58, which may be secured within the catheter hub 52 and may extend distally from the distal end 54 of the catheter hub 52. In some embodiments, the catheter assembly 50 may be a PIVC or any other suitable catheter device.

In some embodiments, the catheter assembly 50 may include or correspond to any suitable catheter assembly 50. In some embodiments, the catheter assembly 50 may be integrated and include an extension tube 60, which may extend from and be integrated with a side port 59 of the catheter hub 52. A non-limiting example of an integrated catheter assembly is the BD NEXIVA^{™} Closed IV Catheter system, available from Becton Dickinson and Company. In some embodiments, a proximal end of the extension tube 60 may be coupled to an adapter, such as, for example, a Y-adapter 70. In some embodiments, the flow restriction device 110 may be fluidly coupled to the Y-adapter 70, such as to a needle free connector 72.

In some embodiments, the catheter assembly 50 may be non-integrated and may not include the extension tube 60. In these and other embodiments, the flow restriction device 110 may be configured to couple to the proximal end 56 of the catheter hub 52 or another suitable portion of the catheter assembly 50. In some embodiments, the flow restriction device 110 may be coupled directly to the catheter assembly 50, eliminating the extension tube 60 and providing a compact catheter system.

In accordance with some embodiments, the flow restriction device 110 may be configured to couple to a blood collection device 40. For example, the flow restriction device 110 may include a female luer connector 113 at proximal housing 112, which may be coupled with a male luer connector 42 of the blood collection device 40. In some embodiments, the blood collection device 40 may be a LLAD.

FIG. 2 illustrates an exploded perspective view of the flow restriction device 110, in accordance with some embodiments of the present disclosure. As depicted in FIG. 2, in some embodiments, the flow restriction device 110 may include a proximal housing 112 configured to couple to the blood collection device 40. For example, the proximal housing 112 may be integrated with the blood collection device 40 or monolithically formed with the blood collection device 40 as a single unit. As another example, the proximal housing 112 may include a female luer connector 113, which may be coupled with a male luer connector 42 of the blood collection device 40.

In some embodiments, the flow restrictor device 110 may be in the form of a cylindrical body extending from the proximal housing 112 to the distal housing 114, with the intermediate housing 116 interposed between the proximal and distal housings 112 and 114. An internal flow channel 170 may be disposed through the proximal, intermediate and distal housings 112, 116, 114, providing a fluid flow pathway from end to end of the flow restrictor device 110.

The distal housing 114 may include a male luer connector 115 and a fluid shield assembly 120 coupled to the distal housing 114. The fluid shield assembly 120 may include a split septum 122 coupled to a luer enclosure 124. For example, the split septum 122 may be glued, welded, overmolded or any other suitable coupling to the luer enclosure 124. The luer enclosure 124 is coupled to the distal housing 114. The luer enclosure 124 may be removably coupled to the distal housing 114, such as by a friction fit. As another example, the luer enclosure 124 may be integrally coupled to the distal housing 114 (e.g., glued, welded, overmolded).

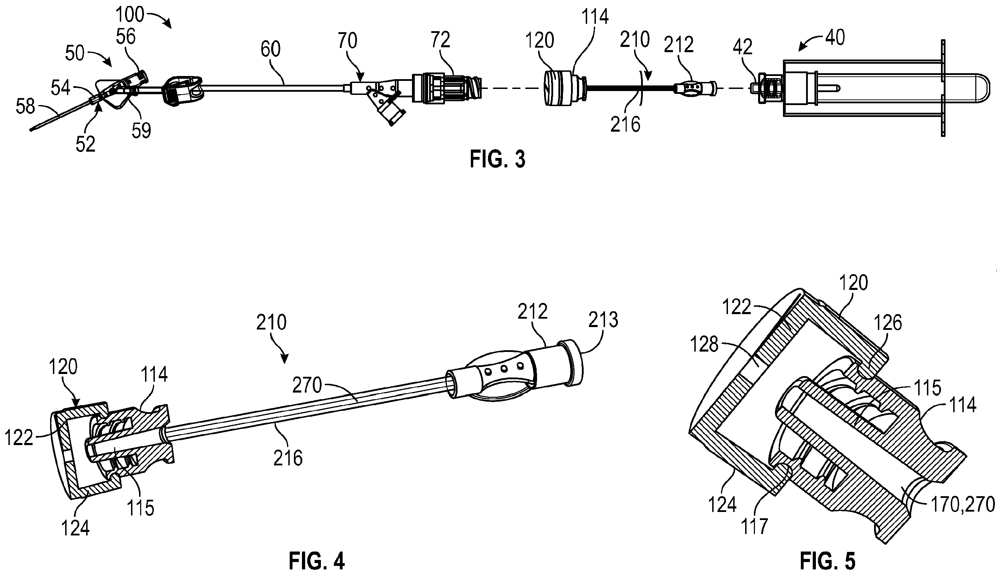

FIG. 3 illustrates the vascular access device 100 including a PIVC assembly 50 that includes a flow restriction device 210 (e.g., an extension set), in accordance with some embodiments of the present disclosure. The flow restriction device 210 may be configured to reduce a likelihood of hemolysis during blood collection using the vascular access device 100. In some embodiments, the flow restriction device 210 may include the distal housing 114, male luer connector 115 and fluid shield assembly 120 described above. In some embodiments, the flow restriction device 210 may be fluidly coupled to the Y-adapter 70. In some embodiments, the flow restriction device 210 may be configured to couple to the proximal end 56 of the catheter hub 52 or another suitable portion of the catheter assembly 50. In some embodiments, the flow restriction device 210 may be coupled directly to the catheter assembly 50, eliminating the extension tube 60 and providing a compact catheter system.

In accordance with some embodiments, the flow restriction device 210 may be configured to couple to a blood collection device 40. For example, the flow restriction device 210 may include a female luer connector 213 at proximal housing 212, which may be coupled with a male luer connector 42 of the blood collection device 40.

FIG. 4 illustrates a partial cross-sectional perspective view of the flow restriction device 210, in accordance with some embodiments of the present disclosure. As depicted in FIG. 4, in some embodiments, the flow restriction device 210 may include a proximal housing 212 configured to couple to the blood collection device 40. For example, the proximal housing 212 may be integrated with the blood collection device 40 or monolithically formed with the blood collection device 40 as a single unit. As another example, the proximal housing 212 may include a female luer connector 213, which may be coupled with a male luer connector 42 of the blood collection device 40.

In some embodiments, the flow restrictor device 210 may be in the form of an IV tube 216 extending from the proximal housing 212 to the distal housing 114, with the IV tube 216 interposed between the proximal and distal housings 212 and 114. An internal flow channel 270 may be disposed through the proximal housing 112, the IV tube 216 and the distal housing 114, providing a fluid flow pathway from end to end of the flow restrictor device 210.

The distal housing 114 may include the male luer connector 115 and the fluid shield assembly 120 may be coupled to the distal housing 114 of flow restrictor device 210. The fluid shield assembly 120 may be removably or integrally coupled to the distal housing 214 in any suitable manner as described above.

FIG. 5 illustrates the fluid shield assembly 120 coupled to the distal housing 114/male luer connector 115 of either flow restrictor device 110 or flow restrictor device 210. The fluid shield assembly 120 may include an engagement rib 126 sized and shaped to be received by a groove 117 of the distal housing 114. The luer enclosure 124 and/or the engagement rib 126 may be formed of a flexible material configured to allow the enclosure to be pushed onto the distal housing 114 until the engagement rib 126 slides into the groove 117. Thus, the fluid shield assembly 120 may be added to or removed from the distal housing 114 as needed.

In some embodiments, the fluid shield assembly 120 may be affixed to the distal housing 114. For example, an adhesive or solvent may be placed in the groove 117 prior to sliding the engagement rib 126 into the groove 117. As another example, the luer enclosure 124 and the engagement rib 126 may be overmolded onto the distal housing 114, where the engagement rib 126 is molded into the groove 117. Here, a separate split septum 122 may be glued, welded, overmolded or any other suitable coupling to the luer enclosure 124. In yet another example, the entire fluid shield assembly 120 may be overmolded onto the distal housing 114. As another example, the engagement rib 126 may be welded (e.g., sonic welding, heat welding) into the groove 117.

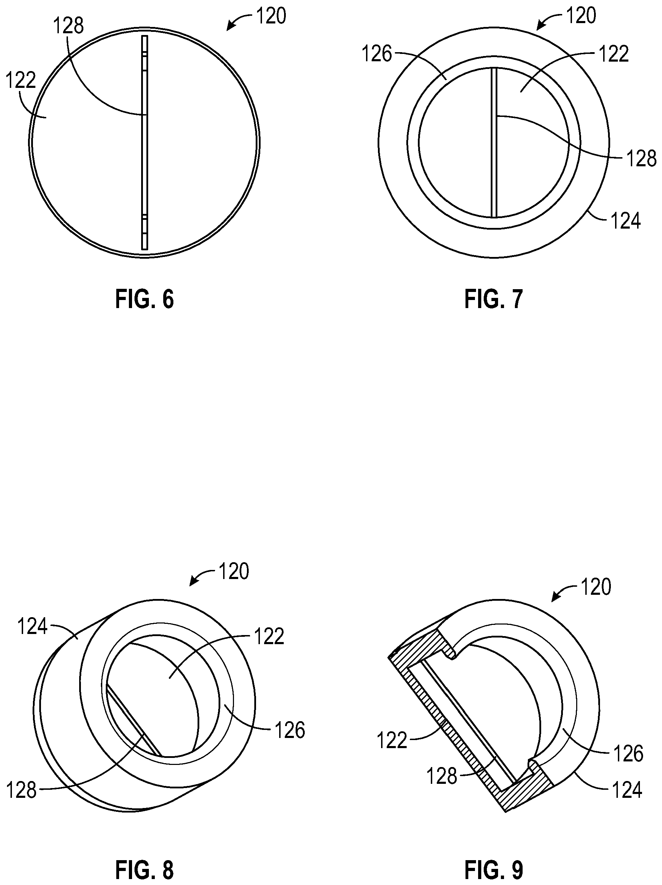

As shown in FIGS. 6-9, the fluid shield assembly 120 may be cylindrical shaped to match the cylindrical shape of a connector (e.g., distal housing 114/male luer connector 115). In aspects of the disclosure, the fluid shield assembly 120 may be sized and shaped to conform with the size/shape of any suitable connector (e.g., oval shaped). The split septum 122 may include a slit 128 and may be formed of any suitable flexible material (e.g., silicone).

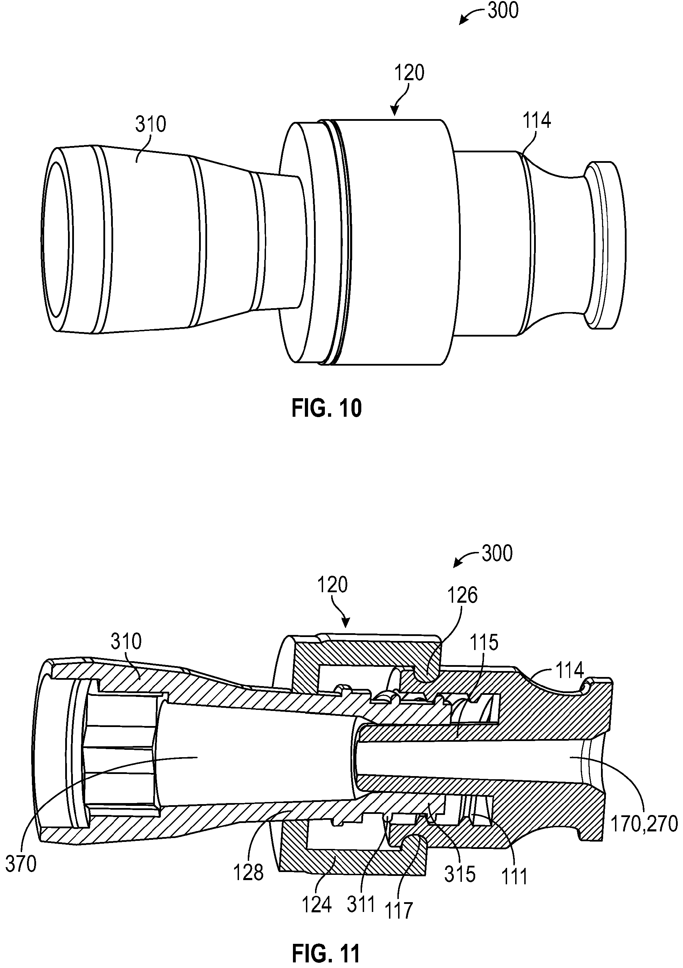

As shown in FIGS. 10 and 11, a connector assembly 300 includes a female connector 310 coupled to the fluid shield assembly 120 and distal housing 114/male luer connector 115 of either flow restrictor device 110 or flow restrictor device 210 shown in FIG. 5. Here, the male luer connector 115 is received within a female luer connector 315 so that the internal flow channel 170, 270 of flow restriction device 110, 210 is fluidly coupled to an internal flow channel 370 of the female connector 310. Also, threads 111 of the distal housing 114/male luer connector 115 are rotatingly engaged with threads 311 of the female connector 310, thus providing a secure connection during use of the connector assembly 300. As can be seen, the slit 128 of the fluid shield assembly 120 has stretched around the outer surface of the female connector 310. In aspects of the disclosure, the female connector 310 may be any suitable female connector configured to mate with the male luer connector 115, such as needle free connector 72 and proximal end 56 of the catheter hub 52, for example.

In use, the fluid shield assembly 120 is coupled to the distal housing 114 such that an end of the male luer connector 115 is enclosed within the luer enclosure 124 and the split septum 122. Any suitable female connector (e.g., proximal end 56, needle free connector 72, female connector 310) may then be coupled to the male luer connector 115. For example, as shown in FIGS. 10 and 11, an end of the female connector 310 is pushed through the slit 128 of the split septum 122, where the flexible material of the split septum 122 provides for the slit 128 to widen so that the flexible split septum 122 stretches and maintains contact with the female connector 310 while the female connector 310 is coupled with the distal housing 114/male luer connector 115. Here, the female connector 310 and the distal housing 114 may each be threaded (e.g., threads 311, 111) and the complete coupling may entail rotating the female connector 310 relative to the distal housing 114 until at least partially or fully seated, thus providing a secure connection.

When disconnection of the flow restriction device 110, 210 is desired, the female connector 310 is uncoupled from the distal housing 114/male luer connector 115 and then the female connector 310 is pulled out through the stretched slit 128 which then contracts back to or close to the size of the slit 128 prior to being connected to the female connector 310. Thus, any fluid (e.g., blood) remaining in the distal housing 114 and/or the lumen of the male luer connector 115 is contained within the luer enclosure 124 of the fluid shield assembly 120. In some aspects of the disclosure, the split septum 122 may function to wipe the end of the female connector 310 as it passes back through the slit 128, thus wiping any fluid from the surfaces of the female connector 310 so that the fluid remains in the fluid shield assembly 120.

In aspects of the disclosure, the fluid shield assembly 120 avoids blood exposure and/or spillage during disconnection of the female connector 310 from the distal housing 114/male luer connector 115. Thus, no secondary action, such as wiping the connectors, needs to be performed by the clinician as any blood drops will be blocked within the luer enclosure 124 and split septum 122 disposed around the distal housing 114/male luer connector 115. In aspects of the disclosure, the split septum 122 self-seals upon removal of the female connector 310.

According to various embodiments of the present disclosure, a method of manufacturing a flow restriction device with a fluid shield assembly may include molding the luer enclosure 124 to the distal housing 114 so that the engagement rib 126 is molded into the groove 117 and/or a portion of the luer enclosure 124 is molded to the outer surface of the distal housing 114. In aspects of the disclosure, the engagement rib 126 may be affixed to the groove 117 via glue or solvent and/or a portion of the luer enclosure 124 may be affixed to the outer surface of the distal housing 114 via glue or solvent. In aspects of the disclosure, the split septum 122 may then be affixed to the exposed end of the luer enclosure 124 by any suitable method, such as gluing, welding or molding. In aspects of the disclosure, the split septum 122 may be affixed to the luer enclosure 124 first to provide an integral fluid shield assembly 120, which may then be affixed to the distal housing 114 as described above. In aspects of the disclosure, the distal housing 114 and luer enclosure 124 may be molded as a single component, and then the fluid shield assembly 120 may be affixed to the luer enclosure 124. In aspects of the disclosure, the distal housing 114, the luer enclosure 124 and the split septum 122 may be molded as a single component in one or more molding operations.

In operation, the flow restriction device 110, 210 with fluid shield assembly 120 may be connected between the catheter assembly 50 and the blood collection device 40 as shown in FIGS. 1 and 3. As such, during blood collection or withdrawal from a patient, blood may flow from the patient's vein into the catheter assembly 50, through the extension tubing 60, enter the internal flow channel 170, 270 of flow restriction device 110, 210 via the distal housing 114, exit the flow restriction device 110, 210 via the proximal housing 112, and enter the blood collection device 40. Accordingly, during blood collection or withdrawal from the patients, the blood may flow into the blood collection device 40 via the internal flow channel 170, 270 having a minimal diameter.

The flow restriction devices 110, 210 of the various embodiments described herein are advantageous over currently existing blood collection systems. For example, during blood draw with currently existing blood draw devices, blood cells may experience shear stress as they flow from a catheter (e.g., catheter system 50) to a blood draw device (e.g., blood collection device 40). The maximum shear stress may be along the wall of the blood cell, often referred to as wall shear stress. Wall shear stress on blood cells is considered a major source of mechanical damage to blood cells causing hemolysis of the blood cells. In some embodiments, the linear internal flow channel 170, 270 having minimal diameter may facilitate increased flow resistance within the vascular access system 100 to distribute the pressure differential and reduce shear stress experienced by the red blood cells of the blood. For example, the minimized diameter of the internal flow channel 170, 270 may provide increased resistance to flow of the blood and thereby decrease blood flow rate within the flow resistance device 110, 210. Since the decreased blood flow rate causes a reduction in shear stress experienced by the red blood cells of the blood, a risk of hemolysis during blood collection may advantageously be reduced.

In addition, the aforementioned configurations of the flow restriction device 110, 210 in which the fluid shield assembly 120 blocks fluid leakage or spillage from the distal housing 114 is advantageous over other blood collection systems in that it is capable of preventing blood spillage from the flow restriction device 110, 210 during disconnection from the blood collection device 40. For example, with other blood collection systems, the recommended blood draw procedures dictate that the blood draw connector should not be disconnected from blood collection device (e.g., luer lock access device (LLAD)) after completion of a blood draw procedure. If the blood draw connector is disconnected from blood collection device, it may cause potential blood spillage from the blood draw connector channel when it is disconnected from blood collection device. In contrast, the fluid shield assembly 120 of the flow restriction devices 110, 210 of the various embodiments described herein in which the split septum 122 blocks fluid from passing from the distal housing 114 provides for the blood to be contained within the fluid shield assembly 120, thereby preventing blood spillage when the flow restriction device 110, 210 is disconnected from the blood collection device 40 for disposal.

The flow restriction devices and associated blood collection systems of the various embodiments described herein additionally provide further advantages over other blood collection systems. For example, add-on flow restriction devices described herein allow for hemolysis-reduction function to be integrated for PIVC blood draw and reduce hemolysis significantly. Further, the flow restriction devices described herein are compatible with PIVC placement and allow for seamless blood draw at insertion. Furthermore, since the flow restriction devices are an add-on which can be easily incorporated without any changes to existing PIVC, there is minimal impact to clinical setting and operations. Additionally, the flow restriction devices described herein minimize or otherwise eliminate the risk of blood spillage during disconnection from the blood draw device. Furthermore, the flow restriction devices described herein have low blood wastage due to a smaller priming volume as compared with other blood collection systems. In addition, the flow restriction devices described herein require less fill time as compared with currently existing blood collection systems.

## Claims

1. A fluid shield assembly (120) for a male luer connector (115), the fluid shield assembly (120) comprising:
an enclosure (124) configured to couple to an end of the male luer connector (115); and
a split septum (122) coupled to an end of the enclosure (124), the split septum (122) comprising a flexible material with a centrally disposed slit (128),
**characterized by** wherein the fluid shield assembly (120) is configured to provide connection and disconnection of a female connector (310) to the male luer connector (115) through the slit (128) of the split septum (122), and to contain fluid within the enclosure (124) and split septum (122) upon disconnection and withdrawal of the female connector (310) from the male luer connector (115).

2. The fluid shield assembly (120) of Claim 1, wherein the enclosure (124) of the fluid shield assembly (120) comprises an internal engagement rib (126) configured to couple with a groove (117) on an outer surface of the male luer connector (115).

3. A flow restriction device (110, 210), comprising:
a proximal housing (112) configured to couple to a fluid collection device (40);
a distal housing (114) comprising a male connector and configured to couple to a catheter assembly (50);
an intermediate housing (116) interposed between the proximal housing (112) and the distal housing (114);
an internal fluid channel (170) extending transversely through the proximal, intermediate and distal housings (112, 116, 114); and
the fluid shield assembly (120) of claim 1.

4. The flow restriction device (110, 210) of Claim 3, wherein the enclosure (124) of the fluid shield assembly (120) comprises an internal engagement rib (126) configured to couple with a groove (117) on the distal housing (114).

5. The flow restriction device (110, 210) of Claim 4, wherein the engagement rib (126) is affixed to the groove (117) by one of an adhesive and a solvent.

6. The flow restriction device (110, 210) of Claim 3, wherein the enclosure (124) of the fluid shield assembly (120) is welded to the distal housing (114).

7. The flow restriction device (110, 210) of Claim 3, wherein the enclosure (124) of the fluid shield assembly (120) is molded to the distal housing (114).

8. The flow restriction device (110, 210) of Claim 3, wherein the split septum (122) is affixed to the enclosure (124) by one of an adhesive and a solvent.

9. The flow restriction device (110, 210) of Claim 3, wherein the split septum (122) is welded to the enclosure (124).

10. The flow restriction device (110, 210) of Claim 3, wherein the split septum (122) is molded to the enclosure (124).

11. The flow restriction device (110, 210) of Claim 3, wherein the flow restriction device (110, 210) is configured to flow blood from the proximal housing (112) to the distal housing (114) and into the fluid collection device (40), and the fluid collection device (40) comprises a blood collection device.

12. The flow restriction device (110, 210) of Claim 3, wherein the female connector is a needle-free connector (72) of the catheter assembly (50).

13. The flow restriction device (110, 210) of Claim 3, wherein the proximal housing (112), the intermediate housing (116) and the distal housing (114) comprise an integral connector assembly having a female connector (113) at the proximal housing (112) and the male connector (115) at the distal housing (114).

14. The flow restriction device (110, 210) of Claim 3, wherein the intermediate housing (116) comprises an intravenous tube (216) connecting a female connector (213) at the proximal housing (212) and the male connector (115) at the distal housing (114).

15. A blood collection system (100), comprising:
a blood collection device (40);
a catheter assembly (50); and
the flow restriction device (110, 210) of any of claims 3 to 14 fluidly coupled to the blood collection device (40).

## Patentansprüche

1. Eine Fluidschutzanordnung (120) für einen männlichen Luer-Steckverbinder (115), wobei die Fluidschutzanordnung (120) umfasst:
ein Gehäuse (124), das so konfiguriert ist, dass es mit einem Ende des männlichen Luer- Steckverbinders (115) verbunden werden kann;
und
ein geteiltes Septum (122), das mit einem Ende des Gehäuses (124) verbunden ist, wobei das geteilte Septum (122) ein flexibles Material mit einem mittig angeordneten Schlitz (128) umfasst,
**dadurch gekennzeichnet, dass** die Fluidschutzanordnung (120) so konfiguriert ist, dass sie eine Verbindung und Trennung eines weiblichen Verbinders (310) mit dem männlichen Luer-Steckverbinder (115) durch den Schlitz (128) des geteilten Septums (122) ermöglicht und Fluid innerhalb des Gehäuses (124) und des geteilten Septums (122) nach dem Trennen und Herausziehen des weiblichen Verbinders (310) aus dem männlichen Luer-Steckverbinder (115) zurückhält.

2. Die Fluidschutzanordnung (120) nach Anspruch 1, wobei das Gehäuse (124) der Fluidschutzanordnung (120) eine innere Eingriffsrippe (126) umfasst, die so konfiguriert ist, dass sie mit einer Nut (117) an einer Außenfläche des männlichen Luer-Steckverbinders (115) in Eingriff kommt.

3. Eine Durchflussbegrenzungsvorrichtung (110, 210), umfassend:
ein proximales Gehäuse (112), das so konfiguriert ist, dass es mit einer Fluidsammelvorrichtung (40) gekoppelt werden kann;
ein distales Gehäuse (114), das einen Verbinder umfasst und so konfiguriert ist, dass es mit einer Katheteranordnung (50) gekoppelt werden kann;
ein Zwischengehäuse (116), das zwischen dem proximalen Gehäuse (112) und dem distalen Gehäuse (114) angeordnet ist;
einen inneren Flüssigkeitskanal (170), der sich quer durch das proximale, das Zwischen- und das distale Gehäuse (112, 116, 114) erstreckt; und
die Fluidschutzanordnung (120) nach Anspruch 1.

4. Die Durchflussbegrenzungsvorrichtung (110, 210) nach Anspruch 3, wobei das Gehäuse (124) der Fluidschutzanordnung (120) eine innere Eingriffsrippe (126) umfasst, die so konfiguriert ist, dass sie mit einer Nut (117) am distalen Gehäuse (114) gekoppelt werden kann.

5. Die Durchflussbegrenzungsvorrichtung (110, 210) nach Anspruch 4, wobei die Eingriffsrippe (126) mit einem Klebstoff oder einem Lösungsmittel an der Nut (117) befestigt ist.

6. Die Durchflussbegrenzungsvorrichtung (110, 210) nach Anspruch 3, wobei das Gehäuse (124) der Fluidschutzanordnung (120) mit dem distalen Gehäuse (114) verschweißt ist.

7. Die Durchflussbegrenzungsvorrichtung (110, 210) nach Anspruch 3, wobei das Gehäuse (124) der Fluidschutzanordnung (120) an das distale Gehäuse (114) angeformt ist.

8. Die Durchflussbegrenzungsvorrichtung (110, 210) nach Anspruch 3, wobei das geteilte Septum (122) mit einem Klebstoff oder einem Lösungsmittel an dem Gehäuse (124) befestigt ist.

9. Die Durchflussbegrenzungsvorrichtung (110, 210) nach Anspruch 3, wobei das geteilte Septum (122) an das Gehäuse (124) verschweißt ist.

10. Die Durchflussbegrenzungsvorrichtung (110, 210) nach Anspruch 3, wobei das geteilte Septum (122) an das Gehäuse (124) angeformt ist.

11. Die Durchflussbegrenzungsvorrichtung (110, 210) nach Anspruch 3, wobei die Durchflussbegrenzungsvorrichtung (110, 210) so konfiguriert ist, dass Blut vom proximalen Gehäuse (112) zum distalen Gehäuse (114) und in die Fluidsammelvorrichtung (40) fließt, und wobei die Fluidsammelvorrichtung (40) eine Blutentnahmevorrichtung umfasst.

12. Die Durchflussbegrenzungsvorrichtung (110, 210) nach Anspruch 3, wobei der weibliche Verbinder ein nadelfreier Verbinder (72) der Katheteranordnung (50) ist.

13. Die Durchflussbegrenzungsvorrichtung (110, 210) nach Anspruch 3, wobei das proximale Gehäuse (112), das Zwischengehäuse (116) und das distale Gehäuse (114) eine integrierte Verbindungsanordnung mit einem weiblichen Verbinder (113) am proximalen Gehäuse (112) und dem männlichen Verbinder (115) am distalen Gehäuse (114) umfassen.

14. Die Durchflussbegrenzungsvorrichtung (110, 210) nach Anspruch 3, wobei das Zwischengehäuse (116) einen intravenösen Schlauch (216) umfasst, der einen weiblichen Verbinder (213) am proximalen Gehäuse (212) und den männlichen Verbinder (115) am distalen Gehäuse (114) verbindet.

15. Blutentnahmesystem (100), umfassend:
eine Blutentnahmevorrichtung (40);
eine Katheteranordnung (50); und
die Durchflussbegrenzungsvorrichtung (110, 210) gemäß einem der Ansprüche 3 bis 14, die fluidisch mit der Blutentnahmevorrichtung (40) verbunden ist.

## Revendications

1. Ensemble de protection de fluide (120) pour un connecteur Luer mâle (115), l'ensemble de protection de fluide (120) comprenant :
un boîtier (124) configuré pour se coupler à une extrémité du connecteur Luer mâle (115) ; et
un septum fendu (122) couplé à une extrémité du boîtier (124), le septum fendu (122) comprenant un matériau flexible avec une fente disposée centralement (128),
**caractérisé en ce que** l'ensemble de protection de fluide (120) est configuré pour permettre la connexion et la déconnexion d'un connecteur femelle (310) au connecteur Luer mâle (115) à travers la fente (128) du septum fendu (122), et à contenir le fluide à l'intérieur du boîtier (124) et du septum fendu (122) lors de la déconnexion et du retrait du connecteur femelle (310) du connecteur Luer mâle (115).

2. Ensemble de protection de fluide (120) selon la revendication 1, dans lequel le boîtier (124) de l'ensemble de protection de fluide (120) comprend une nervure d'engagement interne (126) configurée pour s'accoupler avec une rainure (117) sur une surface extérieure du connecteur Luer mâle (115).

3. Dispositif de restriction de débit (110, 210), comprenant :
un boîtier proximal (112) configuré pour s'accoupler à un dispositif de collecte de fluide (40) ;
un boîtier distal (114) comprenant un connecteur mâle et configuré pour s'accoupler à un ensemble de cathéter (50) ;
un boîtier intermédiaire (116) interposé entre le boîtier proximal (112) et le boîtier distal (114) ;
un canal de fluide interne (170) s'étendant transversalement à travers les boîtiers proximal, intermédiaire et distal (112, 116, 114) ; et
l'ensemble de protection de fluide (120) de la revendication 1.

4. Dispositif de restriction de débit (110, 210) selon la revendication 3, dans lequel le boîtier (124) de l'ensemble de protection de fluide (120) comprend une nervure d'engagement interne (126) configurée pour se coupler avec une rainure (117) sur le boîtier distal (114).

5. Dispositif de restriction de débit (110, 210) selon la revendication 4, dans lequel la nervure d'engagement (126) est fixée à la rainure (117) à l'aide d'un adhésif ou d'un solvant.

6. Dispositif de restriction de débit (110, 210) selon la revendication 3, dans lequel le boîtier (124) de l'ensemble de protection de fluide (120) est soudé au boîtier distal (114).

7. Dispositif de restriction de débit (110, 210) selon la revendication 3, dans lequel le boîtier (124) de l'ensemble de protection de fluide (120) est moulé sur le boîtier distal (114).

8. Dispositif de restriction de débit (110, 210) selon la revendication 3, dans lequel le septum fendu (122) est fixé au boîtier (124) à l'aide d'un adhésif ou d'un solvant.

9. Dispositif de restriction de débit (110, 210) selon la revendication 3, dans lequel le septum fendu (122) est soudé au boîtier (124).

10. Dispositif de restriction de débit (110, 210) selon la revendication 3, dans lequel le septum fendu (122) est moulé sur le boîtier (124).

11. Dispositif de restriction de débit (110, 210) selon la revendication 3, dans lequel le dispositif de restriction de débit (110, 210) est configuré de manière à permettre l'écoulement du sang du boîtier proximal (112) vers le boîtier distal (114) et dans le dispositif de collecte de fluide (40), ledit dispositif de collecte de fluide (40) comprenant un dispositif de prélèvement sanguin.

12. Dispositif de restriction de débit (110, 210) selon la revendication 3, dans lequel le connecteur femelle est un connecteur sans aiguille (72) de l'ensemble de cathéter (50).

13. Dispositif de restriction de débit (110, 210) selon la revendication 3, dans lequel le boîtier proximal (112), le boîtier intermédiaire (116) et le boîtier distal (114) comprennent un ensemble de connecteurs intégré comportant un connecteur femelle (113) au niveau du boîtier proximal (112) et le connecteur mâle (115) au niveau du boîtier distal (114).

14. Dispositif de restriction de débit (110, 210) selon la revendication 3, dans lequel le boîtier intermédiaire (116) comprend un tube intraveineux (216) reliant un connecteur femelle (213) au niveau du boîtier proximal (212) et le connecteur mâle (115) au niveau du boîtier distal (114).

15. Système de prélèvement sanguin (100), comprenant :
un dispositif de prélèvement sanguin (40) ;
un ensemble de cathéter (50) ; et
le dispositif de restriction du débit (110, 210) de l'une quelconque des revendications 3 à 14, couplé de manière fluide au dispositif de prélèvement sanguin (40).
